# EUROPEAN PATENT APPLICATION

(11) **EP 3 712 163 A1**
(43) Date of publication of application: **23.09.2020**
(21) Application number: 20731368.5
(22) Date of filing: 23.01.2020
(51) Int. Cl.: C07K 7/08, A61K 8/64, A61P 17/00, A61Q 19/02, A61K 38/00

(54) **NOVEL PEPTIDE HAVING SKIN-LIGHTENING ACTIVITY AND USE OF SAME**

(30) Priority: 25.01.2019 KR 20190010176
(71) Applicant: NEOMICS CO., LTD., Bongeunsa-ro, Gangnam-gu, Seoul 06153 (KR)
(72) Inventor: PARK, Min Chul, Seoul, 05238 (KR); KIM, Ji Na, Seoul, 05238 (KR); KANG, Su Jin, Seoul, 05238 (KR)
(74) Representative: Pföstl, Andreas
(86) International application number: PCT/KR2020/001226
(87) International publication number: WO 2020/153819

(57) **Abstract**

The present invention relates to novel polypeptides having a skin whitening activity and use thereof. More particularly, the present invention relates to an isolated polypeptide consisting (essentially) of any one amino acid sequence selected from the group consisting of SEQ ID NO: 1, SEQ ID NO: 3, SEQ ID NO: 5, and SEQ ID NO: 7, a skin whitening cosmetic composition comprising the polypeptide as an active ingredient, or a pharmaceutical composition for preventing or treating a pigmentation disease.

<110>     Curebio. Co., Ltd.

<120>     Novel polypeptides with skin whitening activity and use thereof

<130>     OP19-0211/PCT

<150>     KR 10-2019-0010176
<151>     2019-01-25

<160>     10

<170>     KoPatentIn 3.0

<210>     1
<211>     15
<212>     PRT
<213>     Artificial Sequence

<220>
<223>     amino acid sequence of peptide for skin whitening(AIMP1 18aa -
          32aa)

<400>     1
Ala Asp Gln Ile Ile Glu Tyr Leu Lys Gln Gln Val Ser Leu Leu
  1               5                  10                  15

<210>     2
<211>     45
<212>     DNA
<213>     Artificial Sequence

<220>
<223>     nucleic acid sequence of peptide for skin whitening(AIMP1 18aa -
          32aa)

<400>     2
gcagatcaaa tcattgaata tcttaagcag caagtttctc tactt                        45

<210>     3
<211>     15
<212>     PRT
<213>     Artificial Sequence

<220>
<223>     amino acid sequence of peptide for skin whitening(AIMP1 17aa -
          31aa)

<400>     3
Glu Ala Asp Gln Ile Ile Glu Tyr Leu Lys Gln Gln Val Ser Leu
  1               5                  10                  15

<210>     4
<211>     45
<212>     DNA
<213>     Artificial Sequence

<220>

<223>    nucleic acid sequence of peptide for skin whitening(AIMP1 17aa -
         31aa)

<400>    4
gaggcagatc aaatcattga atatcttaag cagcaagttt ctcta                        45

<210>    5
<211>    20
<212>    PRT
<213>    Artificial Sequence

<220>
<223>    amino acid sequence of peptide for skin whitening(AIMP1 16aa -
         35aa)

<400>    5
Ala Glu Ala Asp Gln Ile Ile Glu Tyr Leu Lys Gln Gln Val Ser Leu
  1               5                  10                  15

Leu Lys Glu Lys
             20

<210>    6
<211>    60
<212>    DNA
<213>    Artificial Sequence

<220>
<223>    nucleic acid sequence of peptide for skin whitening(AIMP1 16aa -
         35aa)

<400>    6
gcagaggcag atcaaatcat tgaatatctt aagcagcaag tttctctact taaggagaaa        60

                                                                         60

<210>    7
<211>    20
<212>    PRT
<213>    Artificial Sequence

<220>
<223>    amino acid sequence of peptide for skin whitening(AIMP1 14aa -
         33aa)

<400>    7
Lys Gly Ala Glu Ala Asp Gln Ile Ile Glu Tyr Leu Lys Gln Gln Val
  1               5                  10                  15

Ser Leu Leu Lys
             20

<210>    8
<211>    60
<212>    DNA

<213>    Artificial Sequence

<220>
<223>    nucleic acid sequence of peptide for skin whitening(AIMP1 14aa -
         33aa)


<400>    8
aagggtgcag aggcagatca aatcattgaa tatcttaagc agcaagtttc tctacttaag        60

                                                                          60



<210>    9
<211>    312
<212>    PRT
<213>    Artificial Sequence

<220>
<223>    amino acid sequence of AIMP1 protein(full length_homo sapiens)


<400>    9
Met Ala Asn Asn Asp Ala Val Leu Lys Arg Leu Glu Gln Lys Gly Ala
1               5                   10                  15

Glu Ala Asp Gln Ile Ile Glu Tyr Leu Lys Gln Gln Val Ser Leu Leu
            20                  25                  30

Lys Glu Lys Ala Ile Leu Gln Ala Thr Leu Arg Glu Glu Lys Lys Leu
        35                  40                  45

Arg Val Glu Asn Ala Lys Leu Lys Lys Glu Ile Glu Glu Leu Lys Gln
    50                  55                  60

Glu Leu Ile Gln Ala Glu Ile Gln Asn Gly Val Lys Gln Ile Pro Phe
65                  70                  75                  80

Pro Ser Gly Thr Pro Leu His Ala Asn Ser Met Val Ser Glu Asn Val
                85                  90                  95

Ile Gln Ser Thr Ala Val Thr Thr Val Ser Ser Gly Thr Lys Glu Gln
            100                 105                 110

Ile Lys Gly Gly Thr Gly Asp Glu Lys Lys Ala Lys Glu Lys Ile Glu
        115                 120                 125

Lys Lys Gly Glu Lys Lys Glu Lys Lys Gln Gln Ser Ile Ala Gly Ser
    130                 135                 140

Ala Asp Ser Lys Pro Ile Asp Val Ser Arg Leu Asp Leu Arg Ile Gly
145                 150                 155                 160

Cys Ile Ile Thr Ala Arg Lys His Pro Asp Ala Asp Ser Leu Tyr Val
                165                 170                 175

Glu Glu Val Asp Val Gly Glu Ile Ala Pro Arg Thr Val Val Ser Gly
            180                 185                 190

Leu Val Asn His Val Pro Leu Glu Gln Met Gln Asn Arg Met Val Ile
        195                 200                 205

Leu Leu Cys Asn Leu Lys Pro Ala Lys Met Arg Gly Val Leu Ser Gln

210                 215                 220

Ala Met Val Met Cys Ala Ser Ser Pro Glu Lys Ile Glu Ile Leu Ala
225                 230                 235                 240

Pro Pro Asn Gly Ser Val Pro Gly Asp Arg Ile Thr Phe Asp Ala Phe
                245                 250                 255

Pro Gly Glu Pro Asp Lys Glu Leu Asn Pro Lys Lys Lys Ile Trp Glu
            260                 265                 270

Gln Ile Gln Pro Asp Leu His Thr Asn Asp Glu Cys Val Ala Thr Tyr
        275                 280                 285

Lys Gly Val Pro Phe Glu Val Lys Gly Lys Gly Val Cys Arg Ala Gln
    290                 295                 300

Thr Met Ser Asn Ser Gly Ile Lys
305                 310


<210>    10
<211>    15
<212>    PRT
<213>    Artificial Sequence

<220>
<223>    peptide fragment from AIMP1(AIMP1 32aa - 46aa)


<400>    10
Leu Lys Glu Lys Ala Ile Leu Gln Ala Thr Leu Arg Glu Glu Lys
  1               5                  10                  15

## Description

### TECHNICAL FIELD

This application claims priority from Korean Patent Application No. 10-2019-0010176, filed on January 25, 2019, the entire contents of which are incorporated herein by reference.

The present invention relates to novel polypeptides having a skin whitening activity and use thereof. More particularly, the present invention relates to an isolated polypeptide consisting (essentially) of any one amino acid sequence selected from the group consisting of SEQ ID NO: 1, SEQ ID NO: 3, SEQ ID NO: 5, and SEQ ID NO: 7, a skin whitening cosmetic composition comprising the polypeptide as an active ingredient, or a pharmaceutical composition for preventing or treating a pigmentation disease.

### BACKGROUND OF THE INVENTION

The pharmaceutical industry is shifting from the development of natural or chemical synthetic drugs to the development of protein or peptide drugs. Proteins are fundamental to the function and structure of living organisms, which are directly related to disease, making them an important research subject in the development of therapeutics. In addition, protein or peptide drugs using these drugs are evaluated as an innovative field of medicines because they have fewer side effects and faster efficacy than synthetic drugs. Currently, the importance of biopharmaceuticals is increasing in the pipeline of major pharmaceutical companies, but there are major technical problems until the release of certain biopharmaceuticals such as peptides. For example, low delivery rates of peptide drugs to target sites and long-chain peptide synthesis are obstacles to commercialization. In general, a peptide is composed of about 50 amino acids or less, and in order to succeed as a peptide drug, it is to find a short sequence and exhibit activity. In other words, it is known to select the smallest unit (motif) having excellent physiological activity from full length proteins. Long peptide lengths are known to be expensive to synthesize, not easy to manufacture, and have problems with human absorption.

On the other hand, melanin as the main pigment of the skin has an important effect on health and appearance. The produced melanin migrates to keratinocytes, and is known to have a photoprotective effect of absorbing and blocking excessive ultraviolet rays. However, it has been reported that overproduction and/or accumulation of melanin leads to skin diseases such as blemishes and freckles, and can lead to skin cancer in severe cases.

The production of melanin and the resulting pigmentation process involves the action of an enzyme called tyrosinase, followed by a series of oxidation processes that produce an excess of polymer called melanin and accumulate in the skin. The tyrosinase is the most important enzyme in the process of blackening of the skin (particularly, pigment excess accumulation), which makes tyrosine, an amino acid, into dopa (DOPA, 3,4-dihydroxyphenylalanine) and/or dopaquinone (DOPA-quinone, pheomelanin), an intermediate of melanin polymer production. As described above, the action of tyrosinase is absolute in the process of producing melanin. Therefore, it is possible to regulate melanin production by inhibiting the expression of the enzyme protein or inhibiting the activity of the enzyme. The investigation for whitening functional materials having these effects is being made (Non Patent Literature 1).

However, in the case of many known materials, a skin whitening effect is insufficient or safety issues for the skin have been raised. For example, arbutin has become a problem for some activities. That is, arbutin is also known to have problems with the cytotoxicity of the hydroquinone compound family. Therefore, the investigation for functional materials of skin whitening having a safe and significant effect is continuously required.

### < Prior technical literatures>

### < Non-Patent Documents>

(Non-Patent Documents 1) Te-Sheng Chang, Natural Melanogenesis Inhibitors Acting Through the Down-Regulation of Tyrosinase Activity, Materials 2012, 5, 1661-1685.

### DETAILED DESCRIPTION OF THE INVENTION

### TECHNICAL PROBLEM

Accordingly, the present inventors have made an effort to find a new skin whitening functional material, and have completed the present invention after they have identified short fragment polypeptides which have a particularly excellent skin whitening function (particularly, the excellent effects of inhibiting melanin production and/or inhibiting tyrosinase), independently of the function known for the AIMP1 protein.

Therefore, an aspect of the present invention is directed to provide a cosmetic composition for skin whitening comprising, or consisting (essentially) of a polypeptide consisting of any one amino acid sequence selected from the group consisting of SEQ ID NO: 1, SEQ ID NO: 3, SEQ ID NO: 5, and SEQ ID NO: 7 as an active ingredient.

Another aspect of the present invention is to provide a pharmaceutical composition for preventing or treating a pigmentation disease comprising, or consisting (essentially) of a polypeptide consisting of any one amino acid sequence selected from the group consisting of SEQ ID NO: 1, SEQ ID NO: 3, SEQ ID NO: 5, and SEQ ID NO: 7 as an active ingredient.

Another aspect of the present invention is to provide a use of a polypeptide consisting of any one amino acid sequence selected from the group consisting of SEQ ID NO: 1, SEQ ID NO: 3, SEQ ID NO: 5 and SEQ ID NO: 7 for preparing a cosmetic composition for skin whitening.

Still another aspect of the present invention is to provide a method for whitening the skin of a subject, the method comprising administering to the subject in need thereof an effective amount of a composition comprising a polypeptide consisting of any one amino acid sequence selected from the group consisting of SEQ ID NO: 1, SEQ ID NO: 3, SEQ ID NO: 5 and SEQ ID NO: 7 as an active ingredient.

Still another aspect of the present invention is to provide a use of a polypeptide consisting of any one amino acid sequence selected from the group consisting of SEQ ID NO: 1, SEQ ID NO: 3, SEQ ID NO: 5 and SEQ ID NO: 7 for preparing an agent for treating a pigmentation disease.

Still another aspect of the present invention is to provide a method for treating a pigmentation disease in a subject, the method comprising administering to the subject in need thereof an effective amount of a composition comprising a polypeptide consisting of any one amino acid sequence selected from the group consisting of SEQ ID NO: 1, SEQ ID NO: 3, SEQ ID NO: 5 and SEQ ID NO: 7 as an active ingredient.

### TECHNICAL SOLUTION

An embodiment according to an aspect of the present invention provides a cosmetic composition for skin whitening comprising, or consisting (essentially) of a polypeptide consisting of any one amino acid sequence selected from the group consisting of SEQ ID NO: 1, SEQ ID NO: 3, SEQ ID NO: 5, and SEQ ID NO: 7 as an active ingredient.

An embodiment according to another aspect of the present invention provides a pharmaceutical composition for preventing or treating a pigmentation disease comprising, or consisting (essentially) of a polypeptide consisting of any one amino acid sequence selected from the group consisting of SEQ ID NO: 1, SEQ ID NO: 3, SEQ ID NO: 5, and SEQ ID NO: 7 as an active ingredient.

An embodiment according to another aspect of the present invention provides a use of a polypeptide consisting of any one amino acid sequence selected from the group consisting of SEQ ID NO: 1, SEQ ID NO: 3, SEQ ID NO: 5 and SEQ ID NO: 7 for preparing a cosmetic composition for skin whitening.

An embodiment according to another aspect of the present invention provides a method for whitening the skin of a subject, the method comprising administering to the subject in need thereof an effective amount of a composition comprising, or consisting (essentially) of a polypeptide consisting of any one amino acid sequence selected from the group consisting of SEQ ID NO: 1, SEQ ID NO: 3, SEQ ID NO: 5 and SEQ ID NO: 7 as an active ingredient.

An embodiment according to still another aspect of the present invention provides a use of a polypeptide consisting of any one amino acid sequence selected from the group consisting of SEQ ID NO: 1, SEQ ID NO: 3, SEQ ID NO: 5 and SEQ ID NO: 7 for preparing an agent for treating a pigmentation disease.

An embodiment according to still another aspect of the present invention provides a method for treating a pigmentation disease in a subject, the method comprising administering to the subject in need thereof an effective amount of a composition comprising, or consisting (essentially) of a polypeptide consisting of any one amino acid sequence selected from the group consisting of SEQ ID NO: 1, SEQ ID NO: 3, SEQ ID NO: 5 and SEQ ID NO: 7 as an active ingredient.

Hereinafter, the present invention will be described in detail.

In the present invention, the term 'comprising' is used in the same way as 'containing' or 'characterized by', and does not exclude additional component elements or method steps not mentioned in the composition or method. The term 'consisting of' is used in the same manner as 'composed of' and means to exclude additional elements, steps, or components, which are not separately described. The term "consisting essentially of" means in the scope of the composition or method, including the component elements or steps described, as well as the component elements or steps that do not substantially affect their basic properties.

As used herein, the term "polypeptides" or "proteins" are used according to the conventional meaning, and refer to a polymer of amino acid (aa) residues as generally found in nature. Polypeptides are not limited to a particular length, but in the context of the present invention generally represent some fragments of a full length protein. They may include post-translational regulations, such as glycosylation, acetylation, and phosphorylation, and other modifications known in the art (naturally occurring and non-naturally occurring regulations). Polypeptides and proteins of the invention can be prepared using any of a variety of known recombinant and/or synthetic techniques, and the examples are described further below.

As used herein, the one letter (three letters) of amino acid means the following amino acids according to standard abbreviation provisions in the field of biochemistry:
A (Ala): alanine; C (Cys): cysteine; D (Asp): aspartic acid; E (Glu): glutamic acid; F (Phe): phenylalanine; G (Gly): glycine; H (His): histidine; I (Ile): isoleucine; K (Lys): lysine; L (Leu): leucine; M (Met): methionine; N (Asn): asparagine; O (Ply): pypyrrolysine; P (Pro): proline; Q (Gln): glutamine; R (Arg): arginine; S (Ser): serine; T (Thr): threonine; U (Sec): selenocysteine; V (Val): valine; W (Trp): tryptophan; Y (Tyr): tyrosine.

In addition, the amino acid sequences of all peptides herein are described from the amino terminus (N-terminal, amino terminal or N-terminal) to the carboxy terminus (C-terminal, carboxy terminal or C-terminal) according to standard definitions in the biochemical field.

Conventionally, it is known that AIMP1 binds to the multi-tRNA synthetase complex to enhance the catalytic activity of the multi-tRNA synthetase. In contrast to these previously known regular activities, the present invention derives from the discovery that certain polypeptides (fragments) derived from aminoacyl tRNA synthetase complex interacting multifunctional protein 1 (AIMP1) possess therapeutically related non-canonical biological activities.

Therefore, the present inventors first identified that among the numerous types of fragment polypeptides that can be prepared from AIMP1 protein, the fragment polypeptides consisting of (or consisting essentially of) any one amino acid sequence selected from the group consisting of SEQ ID NO: 1, SEQ ID NO: 3, SEQ ID NO: 5, and SEQ ID NO: 7 exhibit particularly excellent skin whitening efficacy. In particular, the fragment polypeptides of the present invention corresponded to equal or higher levels compared to arbutin, a representative skin whitening ingredient currently available commercially, having the skin whitening effect such as effects of inhibiting melanin production and/or inhibiting tyrosinase.

Accordingly, the present invention provides an isolated polypeptide, wherein the isolated polypeptide consist of any one amino acid sequence selected from the group consisting of SEQ ID NO: 1, SEQ ID NO: 3, SEQ ID NO: 5 and SEQ ID NO: 7.

As used herein, the term 'isolated' means altered by a human from a natural state. That is, it means changed and/or removed from the natural environment. For example, polynucleotides or polypeptides that are inherently present in an organism are not isolated, but the same polynucleotides or polypeptides isolated from a coexistent substance in its natural state are 'isolated'. Furthermore, polynucleotides or polypeptides introduced into an organism by transformation, genetic modification or any other recombinant method are 'isolated' even if present in the organism.

Polypeptides provided by the present invention are meant to include functional equivalent of the isolated polypeptide, characterized in that consisting of any one amino acid sequence selected from the group consisting of SEQ ID NO: 1, SEQ ID NO: 3, SEQ ID NO: 5 and SEQ ID NO: 7 described above. "Functional equivalent" refers to a polypeptide having at least 70% or more, preferably 80% or more, more preferably 90% or more sequence homology (or identity) with the amino acid sequence of the polypeptide of the present invention. For example, it includes polypeptides having 70%, 71%, 72%, 73%, 74%, 75%, 76%, 77%, 78%, 79%, 80%, 81%, 82%, 83%, 84%, 85 %, 86%, 87%, 88%, 89%, 90%, 91%, 92%, 93%, 94%, 95%, 96%, 97%, 98%, 99% and 100% sequence homology, and refers to the polypeptides that exhibit substantially the same physiological activity as the polypeptides of the present invention. Here, the term "the physiological activity of substantially homogeneous" refers to a state that exhibits a whole or about the same degree of a particular property. Specifically, the term "the physiological activity of substantially homogeneous" as used herein refers to the activities of a skin whitening, anti-pigmentation (prevention), pigmentation inhibition (improvement and treatment), as well as the effects of inhibiting melanin production and/or inhibiting tyrosinase.

Such functional equivalent may be those in which some of the amino acid sequences of the polypeptides of the invention are the result of additions, substitutions or deletions. Substitution of amino acids in the above is preferably a conservative substitution. Examples of conservative substitutions of naturally occurring amino acids are as follows: aliphatic amino acids (Gly, Ala, Pro), hydrophobic amino acids (Ile, Leu, Val), aromatic amino acids (Phe, Tyr, Trp), acidic amino acids (Asp) , Glu), basic amino acids (His, Lys, Arg, Gln, Asn) and sulfur containing amino acids (Cys, Met). The functional equivalent also includes variants in which some of the amino acids are deleted on the amino acid sequence of the polypeptide of the present invention. Deletion or substitution of these amino acids is preferably located in a region not directly related to the physiological activity of the polypeptides of the present invention. Also, there are variants in which some amino acids are added to both the ends or within the sequences of the amino acid sequences of the polypeptides of the present invention.

In one example, the functional equivalent is isolated polypeptides, wherein they consist essentially of any one amino acid sequence selected from the group consisting of SEQ ID NO: 1, SEQ ID NO: 3, SEQ ID NO: 5, and SEQ ID NO: 7.

The scope of functional equivalents of the present invention also includes polypeptide derivatives in which some chemical structures of the protein are modified while maintaining the basic backbone of the protein and its physiological activity. For example, this includes structural modifications for altering the stability, hypotonicity, volatility or solubility of the polypeptides of the present invention.

Sequence homology and homogeneity herein is defined as the percentage of amino acid residues in the candidate sequence relative to the amino acid sequence of the polypeptide of the present invention after aligning the amino acid sequence and the candidate sequence of the polypeptides of the present invention and introducing a gap. If necessary, conservative substitutions as part of sequence homogeneity are not considered in order to obtain maximum percentage of sequence identity. In addition, the N-terminus, C-terminus or internal extension, deletion or insertion of the amino acid sequences of polypeptides of the present invention may not be interpreted as sequences affecting sequence homology or homogeneity. In addition, the sequence homogeneity can be determined by common standard methods used to compare similar portions of the amino acid sequences of two proteins or polypeptides. BLAST or such a computer program aligns two proteins or polypeptides so that each amino acid is optimally matched (along the full length sequence of one or two sequences or along the predicted portion of one or two sequences). The program provides a default opening penalty and a default gap penalty, and provides a scoring matrix such as PAM250 (Standard scoring matrix; Dayhoff et al., In Atlas of Protein Sequence and Structure, vol 5, supp 3, 1978) that can be used in conjunction with a computer program. For example, the percentage homogeneity can be calculated as follows. The total number of identical matches is multiplied by 100 and then divided by the sum of the length of the longer sequence in the matched span and the number of gaps introduced into the longer sequence to align the two sequences.

Polypeptides of the present invention can be prepared using available techniques known in the art.

In one example, it may be prepared using any of a variety of proteases. As examples, there may be achromopeptidase, aminopeptidase, ancrod, angiotensin converting enzyme, bromelain, calpain, calpain I, calpain II, carboxypeptidase A, carboxypeptidase B, carboxypeptidase G, carboxypeptidase P, carboxypeptidase W, carboxypeptidase Y, caspase 1, caspase 2, caspase 3, caspase 4, caspase 5, caspase 6, caspase 7, caspase 8, caspase 9, caspase 10, caspase 11, caspase 12, caspase 13, cathepsin B, cathepsin C, cathepsin D, cathepsin E, cathepsin G, cathepsin H, cathepsin L, chymopapain, chymase, chymotrypsin, clostripain, collagenase, complement C1r, complement C1s, complement Factor D, complement factor I, cucumisin, dipeptidyl peptidase IV, elastase, leukocyte, elastase, pancreatic, endoproteinase Arg-C, endoproteinase Asp-N, endoproteinase Glu-C, endoproteinase Lys-C, enterokinase, factor Xa, ficin, furin, granzyme A, granzyme B, HIV Protease, IGase, kallikrein tissue, leucine aminopeptidase, general, leucine aminopeptidase, cytosol, leucine aminopeptidase, microsomal, matrix metalloprotease, methionine aminopeptidase, neutrase, papain, pepsin, plasmin, prolidase, pronase E, prostate specific antigen, protease alkalophilic from Streptomyces griseus, protease from Aspergillus, protease from Aspergillus saitoi, protease from Aspergillus sojae, protease B. licheniformis, alkaline or alcalase, protease from Bacillus polymyxa, protease from Bacillus sp, protease from Rhizopus sp., protease S, proteasomes, proteinase from Aspergillus oryzae, proteinase 3, proteinase A, proteinase K, protein C, pyroglutamate aminopeptidase, rennin, streptokinase, subtilisin, thermolysin, thrombin, tissue plasminogen activator, trypsin, tryptase and urokinase. Those skilled in the art can easily determine which protease is appropriate by considering the chemical specificity of the fragment to be produced.

In another example, the polypeptides disclosed herein can be synthesized using any suitable procedure known to those of skill in the art, such as namely known polypeptide synthesis methods (e.g. genetic engineering methods and chemical synthesis). For example, the polypeptide according to the present invention can be prepared by recombinant techniques according to genetic engineering. Preparation of peptides by genetic engineering, for example, first constructs the polypeptides of the present invention or nucleic acids (polynucleotides) encoding its functional equivalents according to conventional methods. The nucleic acid can be prepared by amplification by PCR using appropriate primers. Alternatively, DNA sequences may be synthesized by standard methods known in the art, such as using automated DNA synthesizers. The constructed nucleic acid is operably linked thereto to insert into a vector comprising one or more expression control sequences (e.g., promoters, enhancers, etc.) that regulate expression of the nucleic acid to produce a recombinant expression vector and to transform the host cell. The cell is cultured under media and conditions appropriate for the desired polypeptide to be expressed to recover substantially pure polypeptide expressed from the nucleic acid from the culture. The recovery can be carried out using methods known in the art. The present invention is not limited thereto, but can be separated and purified by methods known in the art, for example, extraction, recrystallization, various chromatography (gel filtration, ion exchange, precipitation, adsorption, and reverse phase), electrophoresis, and countercurrent distribution. As used herein, the term 'substally pure polypeptide' means that the polypeptides according to the present invention dose not substantially include any other protein derived from a host cell.

Also, for example, polypeptides according to the invention can be prepared by chemical synthesis methods known in the art. Representative methods include, but are not limited to, liquid or solid phase synthesis, fragment condensation, F-MOC or T-BOC chem istry.

As a specific example, the polypeptides of the present invention can be prepared by direct peptide synthesis using a solid phase technique. The solid phase peptide synthesis (SPPS) method can initiate synthesis by attaching functional units called linkers to small porous beads to drive the chain of peptides. Unlike the liquid phase method, the peptides covalently bind to the beads to prevent them from falling off by filtration until cleaved by certain reactants such as trifluoroacetic acid (TFA). Synthesis is achieved by repeating the cycles (cycle, deprotection-wash-coupling-wash) of protection, deprotection, and coupling processes between re-emerged amine group and new amino acid, which bind the N-terminal amine and N-protected amino acid unit of the peptide attached to the solid phase. The SPPS method may be performed by using a microwave technology, and the microwave technology may shorten the time required for coupling and deprotection of each cycle by applying heat during the peptide synthesis process. The thermal energy can prevent the expanding peptide chain from folding or forming aggregation, and promote chemical bonding.

It is also possible to prepare a polypeptide of the present invention by liquid phase peptide synthesis, and the specific method of which is referred to the following documents: US Patent No. 5,516,891. In addition, the polypeptide of the present invention can be synthesized by various methods such as mixing the solid-phase synthesis method and the liquid-phase synthesis method, and the method of manufacture is not limited to the means described herein. Protein synthesis can be performed using manual techniques or by automation. Automated synthesis can be accomplished using, for example, an Applied Biosystems 431A Peptide Synthesizer (Perkin Elmer). Alternatively, the various fragments can be chemically synthesized separately and combined using chemical methods to prepare the desired molecule.

In addition, if necessary, the polypeptide of the present invention may be modified by addition of appropriate functional groups such as phosphorylation, sulfation, acrylation, glycosylation, methylation, farnesylation, acetylation, and amidation.

Furthermore, the present invention provides polynucleotides encoding the polypeptides of the present invention as described above.

The term "polynucleotide", "nucleic acid" refers to deoxyribonucleotides (DNA) or ribonucleotides (RNA) in the form of single-stranded or double-stranded. Unless otherwise limited, known analogues of natural nucleotides that hybridize to nucleic acids in a manner similar to naturally occurring nucleotides are also included. Preferably, the terms "DNA", "polynucleotide" and "nucleic acid" as used herein refer to a DNA molecule isolated from the total genomic DNA of a particular species. Thus, a DNA fragment (part, segment) encoding a polypeptide refers to a DNA fragment consisting of one or more coding sequences that are substantially isolated, or purified from the total genomic DNA of the species from which the DNA fragment can be obtained. The terms 'DNA fragment' and 'polynucleotide' include DNA fragments and smaller fragments of the fragments, and also include recombinant vectors (e.g., plasmids, cosmids, phagemids, bactericidal viruses, and viruses).

As recognized by those skilled in the art, polynucleotides may be single-stranded (code sequences or antisense sequences), or may be double-stranded. They may be DNA molecules (genome, cDNA or synthetic) or RNA molecules. That is, the polynucleotide includes not only the nucleotide sequence encoding the polypeptide, but also a complementary sequence to the sequence. The complementary sequence includes not only a perfectly complementary sequence, but also a substantially complementary sequence. Additional coding or non-coding sequences may be present in the polynucleotides of the present invention. Polynucleotides may also be linked to other molecules and/or support materials.

In the present invention, as long as the polynucleotides can encode the above-described polypeptides of the present invention, the base combination (i.e., base sequences) of the polynucleotides is not particularly limited.

In one example, a polypeptide consisting of amino acid sequences represented by SEQ ID NO: 1 of the present invention, for example, may be encoded by a polynucleotide comprising (or consisting of, or consisting essentially of) the nucleotide sequences represented by SEQ ID NO: 2.

In one example, a polypeptide consisting of amino acid sequences represented by SEQ ID NO: 3 of the present invention, for example, may be encoded by a polynucleotide comprising (or consisting of, or consisting essentially of) the nucleotide sequences represented by SEQ ID NO: 4.

In one example, a polypeptide consisting of amino acid sequences represented by SEQ ID NO: 5 of the present invention, for example, may be encoded by a polynucleotide comprising (or consisting of, or consisting essentially of) the nucleotide sequences represented by SEQ ID NO: 6.

In one example, a polypeptide consisting of amino acid sequences represented by SEQ ID NO: 7 of the present invention, for example, may be encoded by a polynucleotide comprising (or consisting of, or consisting essentially of) the nucleotide sequences represented by SEQ ID NO: 8.

The present invention also provides a vector comprising the polynucleotide.

The term "vector" refers to a means for expressing a gene of interest in a host cell. Vectors of the present invention may be conventional cloning vectors or expression vectors. Expression vectors include a signal or leader sequence for membrane targeting or secretion in an addition to expression control sequence such as a promoter, an operator, an initiation codon, a termination codon, a polyadenylation signal, and an enhancer (promoter), and can be produced variously according to the purpose. The polynucleotide sequence according to the present invention may be operably linked to an expression control sequence, and the operably linked gene sequence and the expression control sequence may be included in one expression vector containing a selection marker and a replication origin together. "Operably linked" means that the appropriate molecule is linked in such a way as to enable gene expression when bound to expression control sequences. The "expression control sequence" refers to a DNA sequence that regulates the expression (particularly, transcription and/or translation) of a polynucleotide sequence operably linked in a particular host cell. Such a regulatory sequence includes a promoter for carrying out transcription, any operator sequence for regulating transcription, a sequence encoding a suitable mRNA ribosomal binding site, and a sequence that controls termination of transcription and translation. The vector also includes a selection marker for selecting a host cell containing the vector and a replication origin in the case of a replicable vector.

Vectors that can be used include, but are not limited to, viral vectors such as plasm id vectors, cosmid vectors and bacteriophage vectors, adenovirus vectors, retroviral vectors, and adeno-associated viral vectors. The Vectors that can be used as recombinant vectors can be constructed by engineering plasmids (e.g., pSC101, pGV1106, pACYC177, ColE1, pKT230, pME290, pBR322, pUC8/9, pUC6,pBD9, pHC79, pIJ61, pLAFR1, pHV14, pGEX series, pET series and pUC19), phages (e.g., λgt4λB,λ-Charon, λΔz1 and M13), or viruses (e.g., CMV and SV40) that are often used in the art.

In one example, the recombinant vector can be constructed with prokaryotic or eukaryotic cells as hosts. For example, when the applicable vector is an expression vector and the prokaryotic cell is a host, it is common to include a strong promoter (for example, a pLλ promoter, a trp promoter, a lac promoter, a tac promoter, and a T7 promoter) capable of proceeding transcription, a ribosome binding site for initiation of translation, and a transcription/translation termination sequence. In the case of using a eukaryotic cell as a host, replication origins that operate in eukaryotic cells included in the vector include f1 origin, SV40 origin, pMB1 origin, adeno origin, AAV origin, CMV origin, and BBV origin, but are not limited to. In addition, promoters derived from the genome of mammalian cells (e.g., metallothionine promoters) or promoters derived from mammalian viruses (e.g., adenovirus late promoters, vaccinia virus 7.5K promoters, SV40 promoters, Cytomegalovirus (CMV) promoter and tk promoter of HSV) can be used and generally have a polyadenylation sequence as a transcription termination sequence.

Accordingly, the present invention also provides a transformant transformed with the vector.

Transformation with the vector can be carried out by transformation techniques known to those skilled in the art. For example, it can be introduced into target cells by transient transfection, microinjection, transduction, cell fusion (e.g., PEG-mediated fusion), microprojectile bombardment, electroporation, calcium phosphate (CaPO4) precipitation, calcium chloride (CaCl2) precipitation, liposome-mediated transfection, DEAE dextran-mediated transfection, polybrene-mediated transfection, electroporation, gene gun and other known methods for introducing DNA into cells, but is not limited to.

The term 'transformant' may be used interchangeably with 'host cell', and refers to a prokaryotic or eukaryotic cell including heterologous DNA introduced into the cell by any means (e.g., electroshock, calcium phosphatase precipitation, microinjection, transformation, and viral infection).

In the present invention, the transformants are all kinds of unicellular organisms commonly used in the cloning field. For example, prokaryotic microorganisms of various bacteria, lower eukaryotic microorganisms and insect cells such as yeast, cells derived from higher eukaryotes including plant cells, mammals, etc. may be used as host cells, but are not limited thereto. Since the expression amount and formula of the protein are different depending on the host cell, those skilled in the art can select and use the most suitable host cell. For example, the microorganism used as a transformant in the present invention is *Escherichia coli, Bacillus subtilis, Streptomyces spp., Pseudomonas spp., Proteus mirabilis, Staphylococcus spp.,* and *Agrobacterium tumefaciens,* but are not limited thereto.

In addition, the present invention provides a cosmetic composition for skin whitening comprising a polypeptide consisting of (or consisting essentially of) any one amino acid sequence selected from the group consisting of SEQ ID NO: 1, SEQ ID NO: 3, SEQ ID NO: 5 and SEQ ID NO: 7 as an active ingredient.

Polypeptides according to the present invention have the excellent effects of inhibiting melanin production and/or inhibiting tyrosinase, and very low cytotoxicity. Therefore, the present invention provides a cosmetic composition for skin whitening comprising the polypeptide according to the present invention as an active ingredient.

In the present invention, the 'effect of skin whitening' means not only brightening the skin tone by inhibiting the synthesis of melanin pigments, but also improving skin hyperpigmentation (e.g., freckles and freckles) due to various causes such as ultraviolet rays, hormones or heredity.

The cosmetic composition of the present invention may be prepared in any formulation conventionally prepared in the art, and it may be prepared in the form of a topically or systemically applicable adjuvant commonly used in the field of dermatology by containing a dermatologically acceptable medium or base in addition to the polypeptide according to the present invention.

In addition to the polypeptide according to the present invention, the cosmetic composition of the present invention may further contain fatty substances, organic solvents, solubilizers, thickeners and gelling agents, softeners, antioxidants, suspending agents, stabilizers, foaming agents, fragrances, surfactants, water, ionic or nonionic emulsifiers, fillers, sequestering agents and chelating agents, preservatives, vitamins, blockers, wetting agents, essential oils, dyes, pigments, hydrophilic or lipophilic active agents, lipid vesicles or adjuvants commonly used in the cosmetic or the field of dermatology such as any other ingredients commonly used in the cosmetic. The above ingredients may be introduced in amounts generally used in the field of dermatology.

Formulations of suitable cosmetic compositions may be provided in the form of, for example, solutions, gels, solid or dough anhydrous products, emulsion obtained by dispersing an oil phase in an aqueous phase, suspension, microemulsion, microcapsules, microgranules or ionic (liposomes), a nonionic vesicle dispersant, a cream, skin, lotion, powder, ointment, spray or conceal stick. It may also be prepared in the form of foam or in the form of an aerosol composition further containing a compressed propellant. Products to which the cosmetic composition of the present invention may be added include, but are not limited to, formulations such as skin lotions, essences, nutritional essences, packs, soaps, shampoos, cleansing foams, cleansing lotions, cleansing creams, body lotions, body cleansers, treatments, serum, latex, press powder, loose powders, and eye shadows.

The content of the polypeptide of the present invention contained in the cosmetic composition of the present invention may be contained in the range of 0.00001 to 10% by weight, preferably 0.0001 to 1% by weight based on the total weight of the cosmetic composition, but is not limited thereto. This can be appropriately determined by those skilled in the art in consideration of factors such as the desired skin whitening effect, the degree of application, the type of formulation, and the stability of the polypeptide in the cosmetic composition.

In addition, the present invention provides a pharmaceutical composition for preventing or treating a pigmentation disease comprising a polypeptide consisting of (or consisting essentially of) any one amino acid sequence selected from the group consisting of SEQ ID NO: 1, SEQ ID NO: 3, SEQ ID NO: 5 and SEQ ID NO: 7 as an active ingredient.

The polypeptide according to the present invention provides a whitening pharmaceutical composition comprising the same as an active ingredient because it is not cytotoxic and has the excellent effect of inhibiting melanin production. Therefore, the pharmaceutical composition according to the present invention can be used to improve and alleviate the pathological state of excessive melanin pigmentation, for example, aging/photo aging, sudden hormonal changes such as pregnancy, wound, inflammation, pigmentation due to skin damage and regeneration caused by burns, melasma, freckles, blemish, moles, macule, nevus, age spot, aging surplus, and melanin dermatosis

The term 'pigmentation disease' as used herein may mean a pigmented disease due to melanin overproduction and/or (over) accumulation thereof. The (hyperpigmentation) disease includes all the lesions and symptoms that are progressing in relation to the melanin mechanism in addition to the skin. In one example, the pigmentation disease may be a skin pigmentation disease, but is not limited thereto.

The pigmentation disease is not particularly limited as long as it is known in the art due to the excessive accumulation of melanin, but includes, for example, dyschromatosis symmetrica hereditaria , reticulose pigmentation, chloasma, melisma, freckles, moles (for example, lentigo, brown moles), nevus, pigmentation by drugs (e.g., drugs selected from minocycline, bleomycin, busulfan or zidovudine), pigmentation after inflammation, hyperpigmentation occurring in dermatitis, lentigo senilis (senile spots), sunlight pigmented spot, gravidic chloasma, age spot, skin blemishes, melanin dermatosis, epidermal melanocytic lesions, Cafe's au lait macules, dermal melanocytic lesions, mongolian spot, lentigines, melanoma, lentigo maligna melanoma, superficial spreading melanoma, acral lentiginous melanoma, nodular melanoma, pigment basal cell carcinoma, dermatofibromas, dermoidcyst, keloid and keratoacanthomas.

The nevus includes specifically squamous nevus, pigment nevus, Becker's nevus, nevus silus, nevus of Ota, acquired bilateral nevus of Ota-like macules, nevus of Ito, blue nevus, melanocytic nevus, junctional nevus, compound nevus, intradermal nevus, halo nevus, congenital nevocytic nevus, spitz nevus and dysplastic nevus.

Preferably, the skin pigmentation disease caused by the excessive accumulation of melanin of the present invention may be dyschromatosis symmetrica hereditaria , reticulose pigmentation, chloasma, melasma lentigo senilis, sunlight pigmented spot, gravidic chloasma, melanin dermatosis, freckles, skin blemishes, moles, macule, age spot, nevus, pigmentation by drugs (e.g., drugs selected from minocycline, bleomycin, busulfan or zidovudine), pigmentation after inflammation, hyperpigmentation occurring in dermatitis.

The pharmaceutical composition according to the present invention is not only directly provided in the form in which the polypeptide of the present invention is included as such, but also provided in an indirect form that the presence and the function of the polypeptide of the present invention is guaranteed when the composition is administered to a cell. In this indirect form, the present invention can provide a pharmaceutical composition for preventing or treating pigmentation diseases comprising a recombinant expression vector comprising a polynucleotide (for example, any one selected from the group consisting of SEQ ID NO: 2, SEQ ID NO: 4, SEQ ID NO: 6 and SEQ ID NO: 8) encoding the polypeptide of the present invention as an active ingredient. Gene (nucleic acid, polynucleotide) delivery using plasmid expression vectors is a method of delivering plasmid DNA directly to mammalian cells, which can be used by humans approved by the FDA (Nabel, E. G., et al., Science, 249:1285-1288, 1990). As the applicable plasmid expression vector in the present invention, mammalian expression plasmids known in the art may be used. For example, pRK5 (European Patent No. 307,247), pSV16B (International Patent Publication No. 91/08291), and pVL1392 (PharMingen) may be included, but are not limited thereto.

The polypeptides according to the invention can be used on their own or in the form of pharmaceutically acceptable salts. In the present invention, "pharmaceutically acceptable" refers to a non-toxic composition that is physiologically acceptable, does not inhibit the action of the active ingredient when administered to humans, and usually does not cause gastrointestinal disorders, allergic reactions such as dizziness or similar reactions. As the salt, an acid addition salt formed by a pharmaceutically acceptable free acid is preferable, and an organic acid and an inorganic acid may be used as the free acid. The organic acid is, but is not limited to, citric acid, acetic acid, lactic acid, tartaric acid, maleic acid, fumaric acid, formic acid, propionic acid, oxalic acid, trifluoroacetic acid, benzoic acid, gluconic acid, metasulfonic acid, glycolic acid, succinic acid, 4-toluenesulfonic acid, glutamic acid and aspartic acid. In addition, the inorganic acid includes, but is not limited to, hydrochloric acid, bromic acid, sulfuric acid and phosphoric acid.

A pharmaceutical composition comprising the polypeptide according to the present invention as an active ingredient can be variously formulated according to the route of administration by a method known in the art together with a pharmaceutically acceptable carrier for the effect of inhibiting melanin biosynthesis and/or skin whitening. The carriers include all kinds of solvents, dispersion media, oil-in-water or water-in-oil emulsions, aqueous compositions, liposomes, microbeads and microsomes.

Pharmaceutically acceptable carriers may further include, for example, oral or parenteral carriers. Oral carriers may include lactose, starch, cellulose derivatives, magnesium stearate, and stearic acid. In addition, carriers for parenteral administration may include water, suitable oils, saline, aqueous glucose and glycols, and may further include stabilizers and preservatives. Suitable stabilizers include antioxidants such as sodium hydrogen sulfite, sodium sulfite or ascorbic acid. Suitable preservatives include benzalkonium chloride, methyl- or propyl-parabens and chlorobutanol. The pharmaceutical composition of the present invention may further include a lubricant, a humectant, a sweetener, a flavoring agent, an emulsifier, and a suspension in addition to the above components. Other pharmaceutically acceptable carriers and agents may be referred to those known in the art.

The total effective amount of the composition of the present invention may be administered to a patient in a single dose, and may be administered by a fractionated treatment protocol which is administered in multiple doses for a long time. The pharmaceutical composition of the present invention may vary the content of the active ingredient depending on the degree of the disease. Although not limited thereto, the preferred total dose of the pharmaceutical composition of the present invention may preferably be about 0.001 µg to 1000 mg, most preferably 0.01 µg to 500 mg per kg of patient body weight per day. However, the dosage of the pharmaceutical composition is determined in consideration of various factors such as the formulation method, route of administration and frequency of treatment, as well as various factors such as the patient's age, weight, health status, sex, severity of the disease, diet and excretion rate. Therefore, those of ordinary skill in the art will be able to determine the appropriate effective dosage of the compositions of the present invention. The pharmaceutical composition according to the present invention is not particularly limited to its formulation, route of administration and method of administration as long as the effect of the present invention is shown.

The composition of the present invention can be administered to any mammal, including humans. As the route of administration, it can be administered orally or parenterally, without limitation. Parenteral administration methods may be, but are not limited to, intravenous, intramuscular, intraarterial, intramedullary, intradural, intracardiac, transdermal, subcutaneous, intraperitoneal, intranasal, intestinal, topical, sublingual or rectal administration. Since the major part of melanin production occurs in the skin, the main route of administration of the pharmaceutical compositions according to the present invention is to be administered transdermally, but is not limited thereto.

The pharmaceutical composition of the present invention may be formulated into an agent for oral or parenteral administration according to the route of administration as described above.

In the case of agents for oral administration, the compositions of the present invention may be formulated using methods known in the art as powders, granules, tablets, pills, dragees, capsules, solutions, gels, syrups, slurries, and suspensions. For example, oral preparations can be obtained by tablets or dragees by combining the active ingredients with solid excipients and then grinding them, adding suitable auxiliaries and then processing them into granule mixtures. Examples of suitable excipients may include a sugar and corn starch including lactose, dextrose, sucrose, sorbitol, mannitol, xylitol, erythritol and maltitol, starches including wheat starch, rice starch and potato starch, celluloses including cellulose, methyl cellulose, sodium carboxymethylcellulose and hydroxypropylmethyl-cellulose rose, and fillers such as gelatin and polyvinylpyrrolidone. In addition, crosslinked polyvinylpyrrolidone, agar, alginic acid or sodium alginate may optionally be added as a disintegrant. Furthermore, the pharmaceutical composition of the present invention may further include an anticoagulant, a lubricant, a humectant, a perfume, an emulsifier and a preservative.

Agents for parenteral administration may be formulated by methods known in the art in the form of injections, creams, lotions, external ointments, oils, moisturizers, gels, aerosols and nasal inhalants. These formulations are described in prescriptions generally known in all pharmaceutical chemistries.

In one example, the pharmaceutical compositions of the present invention may be formulated according to methods known in the art in the form of injections or transdermal agents (including skin external preparations) with suitable parenteral carriers. In this case, but are not limited to, for example, the pharmaceutical composition of the present invention may be prepared by injectable formulation, and lightly pricked the skin with a 30-gauge thin needle, or may be directly applied to the skin. These formulations are described in prescriptions generally known in pharmaceutical chemistry.

In one example, the pharmaceutical compositions of the invention are formulated in an injection. The injections must be sterilized and protected from contamination of microorganisms such as bacteria and fungi. Examples of suitable carriers for injections include, but are not limited to, solvents or dispersion media comprising water, ethanol, polyols (e.g., glycerol, propylene glycol and liquid polyethylene glycols), mixtures thereof and/or vegetable oils. More preferably, suitable carriers may include Hanks' solution, Ringer's solution, phosphate buffered saline (PBS) containing triethanol amine or sterile water for injection, and isotonic solution such as 10% ethanol, 40% propylene glycol and 5% dextrose. In order to protect the injection from microbial contamination, it may further include various antibacterial and antifungal agents such as parabens, chlorobutanol, phenol, sorbic acid and thimerosal. In addition, the injection may in most cases further comprise an isotonic agent such as sugar or sodium chloride.

In one example, the pharmaceutical compositions of the present invention are formulated in the form of transdermal administration. In the case of transdermal administration agents, the forms such as ointments, creams, lotions, gels, externals (liquids), paste, liniments, and aerosols are included. In the above, "transdermal administration" means that the pharmaceutical composition is locally administered to the skin so that an effective amount of the active ingredient contained in the pharmaceutical composition is delivered into the skin.

In one example, the pharmaceutical composition of the present invention may be formulated as external preparations for the skin (liquid medicine for external use). The externals for skin of the present invention may include a polypeptide of the present invention as an active ingredient and may include a pharmaceutically acceptable carrier. In addition, it may further contain fatty substances, organic solvents, solubilizers, thickeners and gelling agents, softeners, antioxidants, suspending agents, stabilizers, foaming agents, fragrances, surfactants, water, ionic or nonionic emulsifiers, fillers, sequestering agents and chelating agents, preservatives, vitamins, blockers, wetting agents, essential oils, dyes, pigments, hydrophilic or lipophilic active agents, lipid vesicles or adjuvants commonly used in the field of dermatology such as any other ingredients commonly used in external preparations for the skin. In addition, the ingredients may be introduced in amounts generally used in the field of dermatology.

The agent forms of the external preparations for the skin are not limited thereto, and examples thereof include liquid unguents, spraying agents, lotions, gels, paste, ointments, aerosols, powders, and transdermal absorption agents. Pharmaceutically acceptable carriers in the external preparations of the present invention may vary depending on the formulation thereof, but may include hydrocarbons such as vaseline, liquid paraffin, and gelled hydrocarbons (plasticis); Animal and vegetable oils such as medium chain fatty acid triglyceride, lard, hard fat, and cacao butter; Higher fatty acid alcohols, fatty acids and esters thereof such as cetanol, stearyl alcohol, stearic acid and isopropyl palmitate; Water-soluble bases such as polyethylene glycol, 1,3-butylene glycol, glycerol, gelatin, white sugar and sugar alcohol; Emulsifiers such as glycerin fatty acid esters, polyoxyl stearic acid and polyoxyethylene-hydrogenated castor oil; Adhesives such as acrylate ester and sodium alginate; Propellants such as liquefied petroleum gas and carbon dioxide; Preservatives such as paraoxy benzoic acid ester. In addition to these, stabilizers, flavors, colorants, pH adjusters, diluents, surfactants, preservatives, and antioxidants can be mixed as necessary. It is preferable to apply the external preparations of the present invention to the affected part of melanin overdeposition by a conventional method.

In addition, the external preparation according to the present invention may be used by adhering on a solid support such as a wound peeling cover of a conventional band-aid. The formulations of this type include, for example, a band-aid (Smith & Nephew Ltd) with a non-stick wound peeling cover in the form of a perforated plastic film; Johnson & Johnson's BAND-AIDs in the form of thin strips, patches, spots, plastic strips; Curity CURAD Ouchless Band-Aid from Colgate-Palmolive Co.(Kendall); And STIK-TITE elastic strips from American White Cross Laboratories Inc. The polypeptide of the present invention can be applied as an active ingredient in this type of formulation.

In addition, the pharmaceutical compositions according to the invention may comprise one or more buffers (e.g., saline or PBS), carbohydrates (e.g., glucose, mannose, sucrose or dextran), antioxidants, bacteriostatic agents, chelating agents (e.g., EDTA or glutathione), adjuvants (e.g., aluminum hydroxide), suspending agents, thickening agents and/or preservatives.

In addition, the pharmaceutical compositions of the present invention may be formulated using methods known in the art to provide rapid, sustained or delayed release of the active ingredient after administration to a mammal.

In addition, the pharmaceutical composition of the present invention may be administered alone or in combination with known compounds having the effects of inhibiting melanin production, inhibiting tyrosinase, or skin whitening.

In addition, the present invention provides a use of a polypeptide consisting of any one amino acid sequence selected from the group consisting of SEQ ID NO: 1, SEQ ID NO: 3, SEQ ID NO: 5 and SEQ ID NO: 7 for preparing a cosmetic composition for skin whitening.

In addition, the present invention provides a method for whitening the skin of a subject, the method comprising administering to the subject in need thereof an effective amount of a composition comprising a polypeptide consisting of any one amino acid sequence selected from the group consisting of SEQ ID NO: 1, SEQ ID NO: 3, SEQ ID NO: 5 and SEQ ID NO: 7 as an active ingredient.

The present invention provides a use of a polypeptide consisting of any one amino acid sequence selected from the group consisting of SEQ ID NO: 1, SEQ ID NO: 3, SEQ ID NO: 5 and SEQ ID NO: 7 for preparing an agent for treating a pigmentation disease.

The present invention is to provide a method for treating a pigmentation disease in a subject, the method comprising administering to the subject in need thereof an effective amount of a composition comprising a polypeptide consisting of any one amino acid sequence selected from the group consisting of SEQ ID NO: 1, SEQ ID NO: 3, SEQ ID NO: 5 and SEQ ID NO: 7 as an active ingredient.

The 'effective amount' of the present invention, when administered to a subject, refers to an amount that has not only an effect of improving, treating, preventing, detecting, diagnosing neutropenia, anemia or osteoporosis, but also an effect of inhibiting or reducing neutropenia, anemia or osteoporosis. The 'subject' may be an animal, preferably an animal including a mammal, especially a human, and may be a cell, tissue, or organ derived from the animal. The subject may be a patient in need of the effect.

The term 'treatment' of the present invention refers generically to ameliorating symptoms of pigmentation disease, or pigmentation disease, which may include treating, substantially preventing, or ameliorating the condition. It includes, but is not limited to, alleviating, healing or preventing one or most of the symptoms resulting from the disease.

### EFFECTS OF THE INVENTION

The polypeptide disclosed in the present invention exhibits excellent tyrosinase inhibitory activity and the inhibitory effect of melanin production, and is not cytotoxic and has excellent potential as a skin whitening functional ingredient.

### BRIEF DESCRIPTION OF THE DRAWINGS

FIG. 1 shows the results confirming the inhibitory capacity of melanin by the polypeptide of the present invention.
FIG. 2 shows the results confirming the inhibitory ability of tyrosinase by the polypeptide of the present invention.
FIG. 3 is a result confirming that the present polypeptide does not exhibit cytotoxicity.

### MODE FOR CARRYING OUT INVENTION

Hereinafter, the present invention will be described in detail.

However, the following examples are illustrative of the present invention, and the present invention is not limited to the following examples.

### Example 1. Preparation of Polypeptides with Whitening Efficacy

The present inventors constructed polypeptide fragments of various lengths at each different location from the full-length AIMP1 protein (SEQ ID NO: 9). From the constructed many fragments of polypeptides, various experiments have been conducted to find polypeptides having short sequence lengths and excellent functionalities. As a result, the present inventors have newly identified that the polypeptide fragments shown in Table 1 are short sequences but have a particularly melanin-reducing effect and tyrosinase inhibitory effect, showing excellent whitening efficacy.

**<Table 1 >**

| Fragment location (Based on SEQ ID NO: 9) | Amino acid sequences of Polypeptides (fragment) having skin whitening efficacy | NO. |
|---|---|---|
| 18aa-32aa | ADQIIEYLKQQVSLL | SEQ ID NO: 1 |
| 17aa-31aa | EADQIIEYLKQQVSL | SEQ ID NO: 3 |
| 16aa-35aa | AEADQIIEYLKQQVSLLKEK | SEQ ID NO: 5 |
| 14aa-33aa | KGAEADQIIEYLKQQVSLLK | SEQ ID NO: 7 |

The excellent skin whitening effect of the polypeptides in the present invention (Table 1) has been demonstrated through experiments using the polypeptide fragments derived from other AIMP1 and known skin whitening components as controls. These results are representatively shown through Example 2 below. 10g Polypeptides with purity 95% or more used in the experiment were made by solid phage synthesis method using F-Moc requested by GL Biochem.

### Example 2. Confirmation of Excellent Skin Whitening Effect of Polypeptides According to the Present Invention

### 2-1. Melanin Assay - Verification of Melanin Inhibitory Effect

B16F10 cells (6x10⁴ cells / well) were aliquoted into 6-well plates and incubated. After 12 hours, the medium was changed to a medium containing α-MSH (Sigma, 0.5 µM) and the test substance (each of the polypeptides or control substances of the present invention). Each 10 µM of polypeptides were treated. In addition, 10 µM of arbutin, which is widely known as a skin whitening ingredient, was used as a control. After incubation for 24 hours, cells were harvested. The number of cells was confirmed using a C-chip (disposable hemacytometer, INCYTO). Cells (1x10⁵ cells) were lysed with 1N NaOH at 70 °C for 1 hour. After centrifugation at 4,000 rpm for 5 minutes, the supernatant was harvested and the melanin content was measured. The absorbance of melanin was measured at 490 nm using a microplate reader (Synergy 2, BioTek).

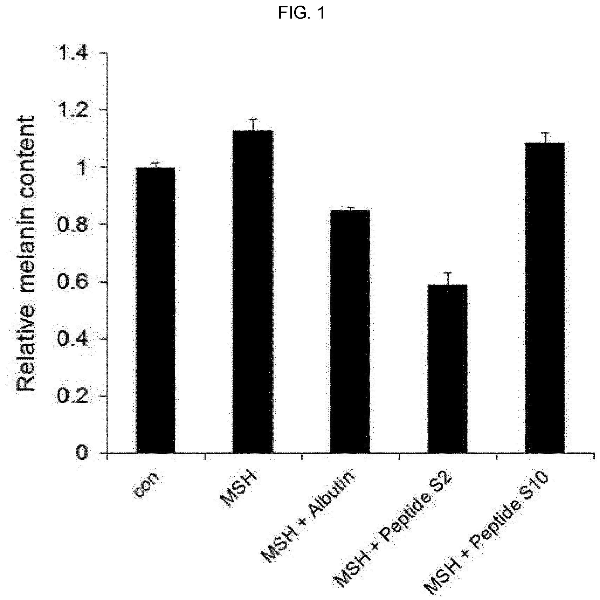

As a result of the experiment, it was confirmed that the polypeptides of the present invention showed remarkable melanin inhibition activity at the same or higher level than arbutin. Experimental results for the polypeptides of a significantly representative example between the experimental group (the present invention) and the control polypeptides are representatively shown in FIG. 1.

Representatively, as shown in FIG. 1, the polypeptide of the present invention (The fragment corresponding to SEQ ID NO: 1 is represented as Peptide S2 in the figure.) showed better inhibitory effect of melanin production than arbutin. On the other hand, in the case of 'LKEKAILQATLREEK (SEQ ID NO: 10, referred to herein as Peptide S10, corresponding to 32aa-46aa based on SEQ ID NO: 9)' which is a polypeptide fragment adjacent to the polypeptide of the present invention, the inhibitory activity of melanin production is completely absent.

### 2-2. Tyrosinase Activity Assay - Verification of Tyrosinase Inhibitory Effect

B16F10 cells (6x10⁴ cells / well) were aliquoted into 6-well plates and incubated. After 12 hours, the medium was changed to a medium containing α-MSH (Sigma, 0.5 µM) and the test substance (each of the polypeptides or control substances of the present invention). Each 10 µM of polypeptides were treated. In addition, 10 µM of arbutin, which is widely known as a skin whitening ingredient, was used as a control. After incubation for 24 hours, cells were harvested. Cells were lysed with PBS buffer containing 1% Triton X-100 (Sigma). After centrifugation at 12,000 rpm for 20 minutes, the supernatant was harvested and the protein concentration was measured by BCA assay kit (Thermo Fisher Scientific). To measure tyrosinase activity, 50 µg of protein (protein contained in the supernatant) was incubated at 37 °C with 5 mM L-DOPA. Absorbance was measured at 490 nm using a microplate reader (Synergy 2, BioTek). Absorbance was measured every 10 minutes for 1 hour.

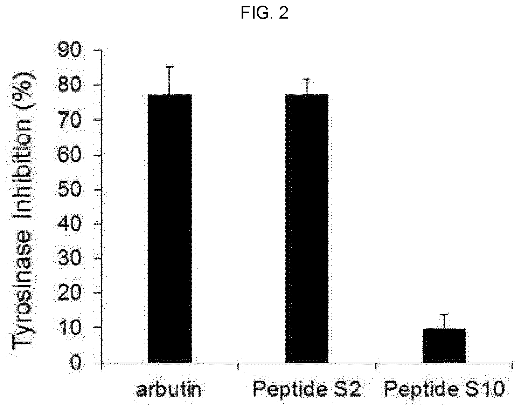

As a result, it was confirmed that the polypeptides of the present invention had the inhibitory activity of tyrosinase at a level equal to or higher than that of arbutin. Experimental results for the polypeptides of a significantly representative example between the experimental group (the present invention) and the control polypeptides are representatively shown in FIG. 2.

As typically shown in FIG. 2, the polypeptide of the present invention (labeled Peptide S2 in the figure corresponding to SEQ ID NO: 1) showed the same level of the inhibitory effect of tyrosinase as arbutin. On the other hand, in the case of 'LKEKAILQATLREEK (SEQ ID NO: 10, herein referred to as Peptide S10, corresponding to 32aa-46aa based on SEQ ID NO: 9)' which is a fragment polypeptide adjacent to the polypeptide of the present invention, the degree of tyrosinase inhibition was insignificant.

### 2-3. Cytotoxicity Assay - Verification of Cytotoxicity

Cell counting kit-8 (CCK-8) was used to measure according to the manufacturer's protocol (Dojindo Laboratories, Japan). CCK-8 assay was used to measure cytotoxicity in starved conditions, the assay is based on the conversion of the water soluble tetrazolium salt, WST-8 (2-(2-methoxy-4-nitrophenyl)-3-(4-nitrophenyl)-5-(2,4-disulfophenyl)-2H-tetrazolium, monosodium salt), into a water soluble formazan dye by reduction with dehydrogenase in the presence of an electron carrier. In serum starved conditions, Vero cells (4x10³ cells / well) were grown in 96-well plates for 12 hours and treated with the respective test materials (polypeptides and control materials of the present invention). Each 10 µM of polypeptides were treated. In the cytotoxicity evaluation, 10 µg / ml of doxorubicin compound was used as a control. After 24 hours, the cells were washed, and the degree of cell growth was evaluated using CCK-8 assay (Dojindo). CCK-8 solution (10 µl) was added to each well and incubated at 37 ° C. for 2 hours. Absorbance was measured at 450 nm using a multiplate reader (Synergy2, Biotek).

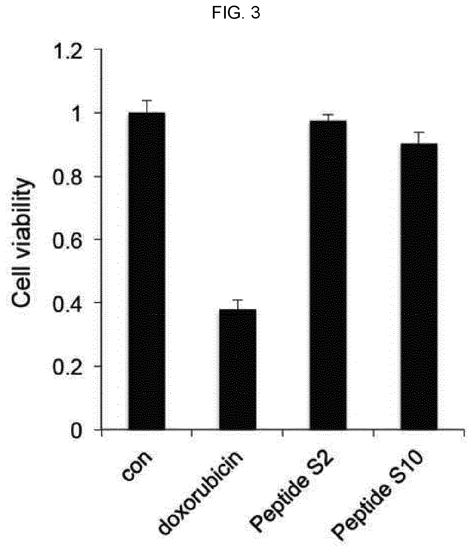

Experimental results confirmed that the polypeptide of the present invention is not cytotoxic. Experimental results for the polypeptides of a significantly representative example between the experimental group (invention) and the control polypeptides are representatively shown in FIG. 3.

### INDUSTRIAL APPLICABILITY

As we have seen above, the present invention relates to novel polypeptides having a skin whitening activity and use thereof. More particularly, the present invention relates to an isolated polypeptide consisting (essentially) of any one amino acid sequence selected from the group consisting of SEQ ID NO: 1, SEQ ID NO: 3, SEQ ID NO: 5, and SEQ ID NO: 7, a skin whitening cosmetic composition comprising the polypeptide as an active ingredient, or a pharmaceutical composition for preventing or treating a pigmentation disease.

The polypeptide disclosed in the present invention exhibits the excellent inhibitory activity of tyrosinase and/or the inhibitory effect of melanin production. It is not cytotoxic and thus has excellent potential as a skin whitening functional ingredient, which is highly likely to be used industrially.

## Claims

1. A cosmetic composition for skin whitening, comprising a polypeptide consisting of any one amino acid sequence selected from the group consisting of SEQ ID NO: 1, SEQ ID NO: 3, SEQ ID NO: 5 and SEQ ID NO: 7 as an active ingredient.

2. The cosmetic composition of claim 1, wherein the polypeptide has an inhibitory activity of melanin production.

3. The cosmetic composition of claim 1, wherein the polypeptide has an inhibitory activity of tyrosinase.

4. A pharmaceutical composition for preventing or treating a pigmentation disease, comprising a polypeptide consisting of any one amino acid sequence selected from the group consisting of SEQ ID NO: 1, SEQ ID NO: 3, SEQ ID NO: 5 and SEQ ID NO: 7 as an active ingredient.

5. The pharmaceutical composition of claim 4, wherein the pigmentation disease is selected from the group consisting of dyschromatosis symmetrica hereditaria , reticulose pigmentation, chloasma, melasma lentigo senilis, sunlight pigmented spot, gravidic chloasma, melanin dermatosis, freckles, skin blemishes, moles, macule, age spot, nevus, pigmentation by drugs, pigmentation after inflammation, and hyperpigmentation occurring in dermatitis.

6. Use of the polypeptide consisting of any one amino acid sequence selected from the group consisting of SEQ ID NO: 1, SEQ ID NO: 3, SEQ ID NO: 5 and SEQ ID NO: 7 for preparing a cosmetic composition for skin whitening.

7. A method for whitening the skin of a subject, the method comprising administering to the subject in need thereof an effective amount of a composition comprising a polypeptide consisting of any one amino acid sequence selected from the group consisting of SEQ ID NO: 1, SEQ ID NO: 3, SEQ ID NO: 5 and SEQ ID NO: 7 as an active ingredient.

8. Use of a polypeptide consisting of any one amino acid sequence selected from the group consisting of SEQ ID NO: 1, SEQ ID NO: 3, SEQ ID NO: 5 and SEQ ID NO: 7 for preparing an agent for treating a pigmentation disease.

9. The use of claim 8, wherein the pigmentation disease is selected from the group consisting of dyschromatosis symmetrica hereditaria, reticulose pigmentation, chloasma, melasma lentigo senilis, sunlight pigmented spot, gravidic chloasma, melanin dermatosis, freckles, skin blemishes, moles, macule, age spot, nevus, pigmentation by drugs, pigmentation after inflammation, and hyperpigmentation occurring in dermatitis.

10. A method for treating a pigmentation disease in a subject, the method comprising administering to the subject in need thereof an effective amount of a composition comprising a polypeptide consisting of any one amino acid sequence selected from the group consisting of SEQ ID NO: 1, SEQ ID NO: 3, SEQ ID NO: 5 and SEQ ID NO: 7 as an active ingredient.

11. The method of claim 10, wherein the pigmentation disease is selected from the group consisting of dyschromatosis symmetrica hereditaria, reticulose pigmentation, chloasma, melasma lentigo senilis, sunlight pigmented spot, gravidic chloasma, melanin dermatosis, freckles, skin blemishes, moles, macule, age spot, nevus, pigmentation by drugs, pigmentation after inflammation, and hyperpigmentation occurring in dermatitis.
